# EUROPEAN PATENT APPLICATION

(11) **EP 4 343 001 A1**
(43) Date of publication of application: **27.03.2024**
(21) Application number: 22382873.2
(22) Date of filing: 22.09.2022
(51) Int. Cl.: C12Q 1/6883, C12Q 1/689

(54) **METHOD FOR THE DIAGNOSIS OF ULCERATIVE COLITIS**

(71) Applicant: Universidade de Santiago de Compostela, 15782 Santiago de Compostela (ES); Servizo Galego De Saúde, 15703 Santiago de Compostela, A Coruna (ES)
(72) Inventor: José Manuel, LEIRO VIDAL, 15782 Santiago de Compostela, A Coruña (ES); Jesús, LAMAS FERNÁNDEZ, 15782 Santiago de Compostela, A Coruña (ES); Rosa Ana, SUEIRO BENAVIDES, 15782 Santiago de Compostela, A Coruña (ES); Iria, BASTÓN REY, 15706 Santiago de Compostela, A Coruña (ES); Manuel, BARREIRO DE ACOSTA, 15706 Santiago de Compostela, A Coruña (ES); Juan Enrique, DOMÍNGUEZ MUÑOZ, 15706 Santiago de Compostela, A Coruña (ES)
(74) Representative: ABG Intellectual Property Law, S.L.

(57) **Abstract**

The invention relates to a method for the diagnosis of ulcerative colitis in a subject by detecting a nucleic acid target region and to oligonucleotides, oligonucleotide probes and kits to be used in this method.

## Description

### FIELD OF THE INVENTION

The present invention belongs to the field of medicine, specifically to the field of diagnosis of ulcerative colitis in a subject with the use of an oligonucleotide probe.

### BACKGROUND OF THE INVENTION

Inflammatory bowel disease (IBD) represents a group of chronic bowel disorders with periods of increased and decreased inflammatory activity. The two most common ones are ulcerative colitis (UC) and Crohn's disease (CD). For those patients with features that make it impossible to distinguish between these two identities, the term unclassifiable inflammatory bowel disease is used. CD is characterized by a patchy transmural involvement that gives rise to different presentations and complications such as the presence of fistulas, abscesses or strictures that can affect any area of the digestive tract. UC, on the other hand, is characterized by a continuous involvement of the mucosa that can be located in the rectum and/or in the rest of the colon. The symptoms that guide us to the diagnosis are very variable but the most frequent clinical picture is characterized by the presence of bloody diarrhea, distension and abdominal pain. Despite having been extensively studied, the etiology of these diseases is still unknown. Genetic as well as immunological and environmental factors have been implicated and are interrelated. The contribution of the intestinal microbiota in the pathogenesis of IBD is nowadays presented as unquestionable, finding different mechanisms involved in the development of inflammation and in its maintenance. Colonization by commensal microorganisms along the gastrointestinal tract begins prenatally, allowing the development and regulation of the immune system, leading to a state of homeostasis between the commensal microbiota and the host. In IBD, an imbalance between the intestinal microbiota and the mucosal immune system has been shown to trigger a chronic inflammatory response.

Due to these limitations in the knowledge of the etiopathogenesis of the disease, as well as in the diagnosis of the different types of IBD, new alternatives for the diagnosis of IBD disorders are required as to allow the right treatment to be provided to the patients.

### BRIEF DESCRIPTION OF THE INVENTION

The inventors have discovered that a particular Bacteroidetes-associated genotype is present in the gut microbiota of subjects suffering from ulcerative colitis but not in healthy subjects, thus allowing to use the detection of said genotype as an early diagnostic tool. The inventors have also developed a probe for the detection of said genotype.

Thus, in a first aspect, the invention relates to an *in vitro* method for the diagnosis of ulcerative colitis in a subject, from here onwards the method of the invention, wherein the method comprises detecting a nucleic acid target region in a sample of feces from the subject, wherein said region comprises the sequence according to SEQ ID NO: 1, the sequence complementary to SEQ ID NO: 1, or a fragment thereof, and wherein the presence of the target region is indicative of the subject having ulcerative colitis.

Another aspect of the present invention relates to an *in vitro* method for the diagnosis of ulcerative colitis in a subject wherein the method comprises detecting a nucleic acid target region in a sample of feces from the subject, wherein said region comprises the sequence according to SEQ ID NO: 9, the sequence complementary to SEQ ID NO: 9, or a fragment thereof, and wherein the presence of the nucleic acid sequence is indicative of the subject having ulcerative colitis.

Another aspect of the present invention relates to an oligonucleotide consisting of a nucleotide sequence according to SEQ ID NO: 4 or SEQ ID NO: 6 or a combination thereof.

A further aspect of the present invention relates to an oligonucleotide probe consisting of a nucleotide sequence according to SEQ ID NO: 1.

Yet another aspect of the present invention relates to a kit comprising the oligonucleotide probe according to the invention and/or the oligonucleotide according to the invention.

One more aspect of the present invention relates to the use of the oligonucleotide according to the invention or the oligonucleotide probe according to the invention or the kit according to the invention for the diagnosis of ulcerative colitis in a subject.

Another aspect of the present invention relates to the use of the oligonucleotide according to the invention or the oligonucleotide probe according to the invention or the kit according to the invention for the detection of *Bacteriodetes* bacteria associated with ulcerative colitis.

### BRIEF DESCRIPTION OF THE DRAWINGS

**Figure 1****.** *Bacteroidetes genotype in faeces and biopsies of ulcerative colitis (UC) patients and healthy controls.*
**Figure 2****.** 2% agarose gel showing the amplicons (arrow) obtained by PCR with the primers FGENC4 / RGENC4 on microbiota DNA isolated from feces of a healthy individual (channels 1 and 2), from an IBD patient (lines 3 and 4) and DNA obtained from *Phocaeicola dorei* (channels 5 and 6). M= 50 bp ladder.
**Figure 3****.** Regression line for qPCR quantification.
**Figure 4****.** Concentration (fg) by qPCR of C4 in controls and patients with UC.
**Figure 5****.** Measure of the accuracy of the test for the set of all possible cut-off points by means of a ROC curve.

### DETAILED DESCRIPTION OF THE INVENTION

The inventors have discovered the association of a specific nucleic acid target region with the disease ulcerative colitis (UC). Said nucleic acid appears to be associated with *Bacteroidetes* bacteria which are present in the gut microbiota of subjects suffering from UC but not in healthy subjects, thus allowing to use the detection of said nucleic acid target region as an early diagnostic tool. In order to use of the discovered new target for UC diagnostics, the inventors have also designed primers and a probe which allows the detection of said nucleic acid sequence.

### Method of the invention

Therefore, a first aspect of the invention relates to an *in vitro* method for the diagnosis of ulcerative colitis in a subject, from here onwards the method of the invention, wherein the method comprises detecting a nucleic acid target region in a sample of feces from the subject, wherein said region comprises the sequence according to SEQ ID NO: 1, the sequence complementary to SEQ ID NO: 1, or a fragment thereof, and wherein the presence of the target region is indicative of the subject having ulcerative colitis.
SEQ ID NO: 1
TGGCATCATGAGTTCACATGTCCGCA

The nucleic acid target region, which belongs to unknown bacteria which comprise in their genome the sequence according to SEQ ID NO: 1, is normally associated with the presence in said bacteria of nucleic acid sequence according to SEQ ID NO: 9 which comprise SEQ ID NO: 1. Therefore, another aspect of the present invention relates to an *in vitro* method for the diagnosis of ulcerative colitis in a subject wherein the method comprises detecting a nucleic acid sequence in a sample of feces from the subject, wherein said region comprises the sequence according to SEQ ID NO: 9, the sequence complementary to SEQ ID NO: 9 or a fragment thereof and wherein the presence of the nucleic acid sequence is indicative of the subject having ulcerative colitis. In a preferred embodiment, the said region comprises the sequence according to SEQ ID NO: 2, the sequence complementary to SEQ ID NO: 2 or a fragment thereof.
SEQ ID NO: 9 
CTTGGCCAGCCTTCTGAAAGGAAGATTAATCCAGGATGGCATCATGAGTTCACATGTCCGCAGATTA
AAGGTATTTTCGGTAGACGATGGGGATGCGTTCCATTAGATAGTAGGCGG
SEQ ID NO: 2

GCCAGCCTTCTGAAAGGAAGATTAATCCAGGATGGCATCATGAGTTCACATGTCCGCAGATTAAAGG
TATTTTCGGTAGACGATGGGGATGCGTTCCATTAGATAGTAGGCGG

In the context of the present invention, *"in vitro* method for the diagnosis of ulcerative colitis" is understood as a method which allows showing the existence of ulcerative colitis in a subject by means of detecting the presence of a nucleic acid target sequence in a sample isolated from the subject.

Diagnosing, as used herein, refers both to the process of attempting to determine and/or identify a possible disease in a subject, i.e., the diagnostic procedure, and to the opinion reached by this process, i.e., the diagnostic opinion. As such, it can also be regarded as an attempt at classification of an individual's condition into separate and distinct categories that allow medical decisions about treatment and prognosis to be made.

In the context of the present invention, the term "diagnosis" relates to the ability to detect and identify the presence of certain bacterial strains in a patient (the subject) that allows showing the existence of ulcerative colitis in a subject. This detection and identification as it is understood by one skilled in the art is not intended to be 100% correct for all the samples. However, it requires that a statistically significant number of diagnosis performed are classified correctly. The amount that is statistically significant can be set by an expert in the field by using different statistical tools, for example, but not limited to, by the determination of confidence intervals, p value determination, Student's t test and discriminating function Fisher. Preferably, the confidence intervals are at least 90%, at least 95%, at least 97%, at least 98% or at least 99%. Preferably, the p value is less than 0.05, 0.01, 0.005 or 0.0001. Preferably, the present invention can correctly detect a disease in at least 60%, at least 70%, by at least 80%, or at least 90% of the subjects of a particular group or population tested.

The term "ulcerative colitis" or its acronym "UC", as used herein, refer to a condition involving inflammation of the colon and rectum. In patients with UC, there is an inflammatory reaction primarily involving the colonic mucosa. The inflammation is typically uniform and continuous with no intervening areas of normal mucosa. Surface mucosal cells as well as crypt epithelium and submucosa are involved in an inflammatory reaction with neutrophil infiltration. Ultimately, this reaction typically progresses to epithelial damage and loss of epithelial cells resulting in multiple ulcerations, fibrosis, dysplasia and longitudinal retraction of the colon.

As used herein, the term "subject" refers to a mammal, and includes but is not limited to domestic and farm animals, primates and humans, for example human beings, non-human primates, cows, horses, pigs, sheep, goats, dogs, cats or rodents. Preferably, the subject is a human, of any age, gender or race. In a particular embodiment the subject is a mammal, preferably a human. In an embodiment, the subject has not taken antibiotics or probiotics within the last 30 days. In another embodiment, the subject has not taken any pharmacological treatment that may alter the intestinal microbiota in the previous 3 months. In another embodiment, the subject does not have a concomitant *Clostridium difficile* or another enteric pathogen infection or any liver disease or malabsorptive disease that alter the intestinal microbiota.

In the present invention, the term "nucleic acid" refers to a repetition of monomers called nucleotides, linked by phosphodiester bonds. There are two types of nucleic acids: DNA (deoxyribonucleic acid) and RNA (ribonucleic acid). In the present invention, "DNA" means the genetic material of living organisms that controls heredity and is located in the nucleus or mitochondria of cells. In the present invention "RNA" means the molecule resulting from the transcription of a DNA sequence. The nucleic acid of the invention may contain one or more modifications to nucleobases, sugars and/or nucleotide bonds.

In the context of the present invention, the term "nucleic acid target region" or "target region" refers to a specific nucleic acid sequence that allows for the unique identification of bacteria which are associated with subjects suffering from UC. Specifically, the target region of the present invention comprises the sequence according to SEQ ID NO: 1, or the sequence complementary to SEQ ID NO: 1, or SEQ ID NO: 2, or the sequence complementary to SEQ ID NO: 2. In a particular embodiment of the method of the invention the target region consists of the nucleotide sequence according to SEQ ID NO: 1 or SEQ ID NO: 2.

In a preferred embodiment of the method of the invention the nucleic acid target region belongs to unknown bacteria, which comprises in its genome the sequence according to SEQ ID NO: 1 or SEQ ID NO: 2. The term "unknown bacteria" as used herein refers to bacteria which despite being able to be detected, normally through bulk PCR reactions with generic primers followed by DNA sequencing of the PCR product, have not been assigned to a specific branch of the "tree of life" and therefore do not have a phylum, genus or species designation. This is mostly due to the incapability of culturing said bacteria and determining their physical attributes.

In a preferred embodiment of the method of the invention the nucleic acid target region belongs to the genome of *Bacteroidetes* bacteria, preferably *Bacteroides* bacteria, more preferably *Phocaeicola dorei,* which comprises in its genome the sequence according to SEQ ID NO: 1 or SEQ ID NO: 2.

The term *"Bacteriodetes"* as used herein refers to the phylum composed of three large classes of Gram-negative, non-spore forming, anaerobic or aerobic, and rod-shaped bacteria that are widely distributed in the environment, including in soil, sediments, and sea water, as well as in the guts and on the skin of animals. Although some *Bacteroidetes* spp. can be opportunistic pathogens, many *Bacteroidetes* are symbiotic species highly adjusted to the gastrointestinal tract. *Bacteroidetes* are highly abundant in intestines.

The term *"Bacteroides"* refers to a genus of Gram-negative and obligate anaerobic bacteria. *Bacteroides* species are non-endospore forming bacilli, and may be either motile or non-motile, depending on the species. In a particular embodiment of the method of the invention the *Bacteroides* bacteria is *Bacteroides dorei* (a.k.a. *Phocaeicola dorei*), preferably a P. *dorei* which comprises in its genome the sequence according to SEQ ID NO: 1 or SEQ ID NO: 2.

In a preferred embodiment of the method of the invention, the nucleic acid target region further comprises the nucleic acid sequence according to SEQ ID NO: 18. In a preferred embodiment of the method of the invention, the nucleic acid target region further comprises the nucleic acid sequences according to SEQ ID NO: 4 and SEQ ID NO: 18.
SEQ ID NO: 4
   GCCAGCCTTCTGAAAGGAAG
SEQ ID NO: 18
   CGTTCCATTAGATAGTAGGCGG

The term "sample", in the context of the present invention, refers to a small quantity or isolated part which is representative of the whole, i.e., whose characteristics are identical to the whole from which the sample is taken. In the context of the present invention the sample is a feces sample. Methods for obtaining said samples, e.g. collection of feces and colonoscopy, are well known in the art and the skilled person in the field would be able to collect said samples without effort.

Several methods are known in the art that can be applied in genotyping, namely restriction fragment length polymorphism identification (RFLPI) of genomic DNA, random amplified polymorphic detection (RAPD) of genomic DNA, amplified fragment length polymorphism detection (AFLPD), DNA sequencing, allele specific oligonucleotide (ASO) probes, and hybridization to DNA microarrays or beads. The act of determining a genotype, or "genotyping" refers to process of determining differences in the genetic make-up, i.e. the genotype, of an individual by examining the individual's DNA sequence using biological assays and comparing it to another individual's sequence or a reference sequence. In a particular embodiment of the method of the invention the detection of the target region is carried out by restriction fragment length polymorphism identification (RFLPI) of genomic DNA, random amplified polymorphic detection (RAPD) of genomic DNA, amplified fragment length polymorphism detection (AFLPD), polymerase chain reaction (PCR), DNA sequencing, allele specific oligonucleotide (ASO) probes, and hybridization to DNA microarrays or beads. Preferably, the detection of the target region is carried out by PCR, more preferably is carried out by PCR and by hybridization with an allele specific oligonucleotide probe.

In another particular embodiment of the method of the invention, from here onwards method I of the method of the invention, the method comprises the steps of:
(i) submitting the nucleic acids obtained from the sample of the subject to an amplification reaction of the nucleic acid target region obtaining an amplification reaction product wherein the amplification reaction product comprises the nucleotide sequence according to SEQ ID NO: 1, or a fragment thereof, and wherein the amplification reaction comprises a forward primer, a reverse primer and an oligonucleotide probe, wherein the forward primer and the reverse primer are specific and capable of amplifying said amplification reaction product, the oligonucleotide probe comprises at least one label capable of emitting a detectable signal and the oligonucleotide probe sequence hybridizes to the nucleotide sequence according to SEQ ID NO: 1 or fragment thereof; and
(ii) detecting the signal emitted by the label during the amplification reaction in step (i),
wherein the detection of the signal emitted by the label is indicative of the subject having ulcerative colitis.

In another particular embodiment of the method of the invention, from here onwards method II of the method of the invention, the method comprises the steps of:
(i) submitting the nucleic acids obtained from the sample of the subject to an amplification reaction of the nucleic acid target region obtaining an amplification reaction product wherein the amplification reaction product comprises the nucleotide sequence according to SEQ ID NO: 1 or a fragment thereof and wherein the amplification reaction comprises a forward primer and a reverse primer wherein the forward primer and the reverse primer are specific and capable of amplifying said target amplification reaction product;
(ii) performing an hybridization reaction of the amplification reaction product with an oligonucleotide probe that hybridizes to the nucleotide sequence according to SEQ ID NO: 1 or a fragment thereof wherein said oligonucleotide probe comprises at least one label capable of emitting a detectable signal, and
(iii) detecting the signal emitted by the label after the hybridization reaction of step (ii),
wherein the detection of the signal emitted by the label is indicative of the subject having ulcerative colitis.

In another particular embodiment of the method of the invention, from here onwards method III of the method of the invention, the method comprises the steps of:
(i) submitting the nucleic acids obtained from the sample of the subject to an amplification reaction of the nucleic acid target region obtaining an amplification reaction product wherein the amplification reaction product comprises the nucleotide sequence according to SEQ ID NO: 1 or a fragment thereof and wherein the amplification reaction comprises a forward primer, and a reverse primer, wherein the forward primer and the reverse primer are specific and capable of amplifying said amplification reaction product; and
(ii) sequencing the amplification reaction product,
wherein the presence in the amplification reaction product of a nucleic acid sequence which comprises SEQ ID NO: 1 is indicative of the subject having ulcerative colitis.

The expression "the nucleic acids obtained from the sample", as used herein, refers to the nucleic acids which were extracted from the samples, i.e., feces, before submitting said nucleic acids to the amplification reaction. Methods to extract nucleic acids from samples, in particular feces samples are well known in the art. Examples of said methods can be found in the "DNA extraction" subheading of the Examples section of the present description.

Once the nucleic acids are obtained from the samples, the first step of the method I, method II and method III of the method of the invention is to submit said nucleic acids to an "amplification reaction", i.e., perform a copy of a region of the nucleic acids, wherein said copy is multiplied exponentially through the use of oligonucleotides, known usually as primers, which are complementary and hybridize with the extremes of said region being amplified.

The different techniques or procedures for carrying out amplification reactions are extensively described in the state of the art, e.g. in Sambrook et al., 2001 (Molecular Cloning A Laboratory Manual. 3rd Edition, Vol. 1, Cold Spring Harbor Laboratory Press, New York). Examples of amplification reactions include, but are not limited to, polymerase chain reaction (PCR) and variations thereof (Regional Amplification Polymerase Chain Reaction (RA-PCR), Real Time Polymerase Chain Reaction (RT-PCR), etc.). In a particular embodiment of the procedure of the invention, the amplification reaction is carried out by means of a polymerase chain reaction (PCR). The protocol followed to carry out a PCR is widely known in the state of the art and commercial kits containing the materials necessary to carry out such amplification are currently available. Likewise, the conditions of temperature, time, reagent concentrations and number of PCR cycles will depend on the DNA polymerase used in the amplification reaction, the specificity of the primers, etc. Thus, in a particular embodiment of the procedure of the invention, the amplification reaction is carried out by a real-time quantitative polymerase chain reaction (PCR) or a real time semi-quantitative polymerase chain reaction (PCR). A Real-Time PCR reaction is basically a conventional PCR in which the amplification equipment (called thermal cyclers) incorporates a fluorescence detection system, the detection being based on the use of specific molecules. The fluorescence can be used to quantify the number of molecules being amplified or to quantify the level of amplification in relation to a reference curve (semi-quantitative). In another embodiment the amplification reaction is carried out by conventional PCR. In another particular embodiment the amplification reaction is carried out by a digital PCR.

Several components in a solution are necessary to carry out an amplification reaction. Thus, in a particular embodiment of the procedure of the invention, the amplification reaction is carried out in the presence of reaction buffer, deoxyribonucleotide triphosphate (dNTP), magnesium ions and DNA polymerase. The term 'reaction buffer' refers to a buffer solution that provides a suitable chemical environment for DNA polymerase activity. The pH of the buffer is usually between 8.0 and 9.5 and is often stabilised with Tris-HCI. A common component of the buffer is potassium ion (K⁺) from KCI, or ammonium sulphate (NH₄)₂SO₄, which promotes primer hybridization. Such a reaction buffer is usually provided commercially in conjunction with DNA polymerase. The term "deoxyribonucleotide triphosphate" or its acronym "dNTP" refers to the triphosphate nucleotides adenine (dATP), cytosine (dCTP), thymine (dTTP) and guanine (dGTP), the bases necessary for DNA construction. The term "divalent ions" as used in the present description refers to chemical elements, in this case ions, which have a valence of two, i.e. they can form up to two bonds. In a particular embodiment, the divalent ion is magnesium (Mg²⁺). In a particular embodiment of the method of the invention the magnesium ions are obtained from magnesium chloride (MgCl₂) present in the reaction mixture at a concentration of 2 to 3 mM. In another particular embodiment of the method of the invention the magnesium ions are obtained from magnesium chloride (MgCl₂) present at a concentration of 2.5 mM in the reaction mixture.

The term 'DNA polymerase' as used in the present description refers to enzymes (E.C.: 2.7.7.7) (cellular or viral) involved in the DNA replication process which carry out the synthesis of the new DNA strand by pairing deoxyribonucleotide triphosphates (dNTPs) with the corresponding complementary deoxyribonucleotides of the template DNA.

The amplification reaction is carried out in specialized equipment called a thermal cycler, which allows multiple cycles of a set of stages with different temperatures and different times per stage. Thus, in a particular embodiment of the method I, method II or the method III of the method of the invention, the amplification reaction conditions comprises the steps:
(i) An initial denaturation step at a temperature between 90°C and 98°C for a time between 1 minutes and 5 minutes;
(ii) A denaturation step at a temperature between 90°C and 98°C for a time between 5 seconds and 60 seconds;
(iii) A hybridization step at a temperature between 50°C and 68°C for between 5 seconds and 45 seconds;
(iv) an extension step at a temperature between 68°C and 76°C for a time between 30 seconds and 90 seconds; and
(v) A final extension step at a temperature between 68°C and 74°C for a time between 1 minutes and 5 minutes,
wherein steps (ii), (iii) and (iv) are repeated at least 20 times, at least 25 times, at least 30 times, at least 32 times, at least 35 times before carrying out step (v).

In a more particular embodiment of the method I, method II or the method III of the method of the invention, the amplification reaction conditions comprises the steps:
(i) An initial denaturation step at a temperature of about 95°C for a time of about 3 minutes;
(ii) A denaturation step at a temperature of about 95°C for a time of about 15 seconds;
(iii) A hybridization step at a temperature of about 59°C for a time of about 15 seconds;
(iv) an extension step at a temperature of about 72°C for a time of about 45 seconds; and
(v) A final extension step at a temperature of about 72°C for a time of about 3 minutes,
wherein steps (ii), (iii) and (iv) are repeated at least 20 times, at least 25 times, at least 30 times, at least 32 times, at least 35 times before carrying out step (v).

The terms "denaturation", "hybridization" and "extension" in the context of the present invention refer to: the separation of the double strands of nucleic acids present in the sample into single strands; the hybridization of primers to the previously formed nucleic acid single strands due to the fact that said primers have a DNA sequence complementary to the target region of the nucleic acids; and extension of a new DNA strand by a DNA polymerase from the 3' end of the primer such that the new strand is complementary to the template strand to which the primer has hybridized.

The amplification reaction of the method I and method II of the method of the invention amplifies the nucleic acid target region (previously defined) which allows the detection and identification of the presence of bacterium which is indicative of the subject having UC. In a particular embodiment of method I or method II of the method of the invention, the amplification reaction amplifies a fragment of the target region of at least 20, at least 30, at least 40, at least 50, at least 60, at least 70, at least 80, at least 90, at least 100 nucleotides in length, wherein said fragment is comprised in the nucleotide sequence according to SEQ ID NO: 1. In a particular embodiment of the method III of the method of the invention, the amplification reaction amplifies a target region of at least 113 nucleotides, 117 nucleotides, at least 120 nucleotides, at least 130 nucleotides, at least 150 nucleotides in which is comprised the sequence according to SEQ ID NO: 2.

In order to amplify the target region the amplification reaction obtains an "amplification reaction product which comprises the nucleotide sequence according to SEQ ID NO: 1 or a fragment thereof". In a particular embodiment of method I, method II and method III of the method of the invention, the amplification reaction product comprises a nucleotide sequence with at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 100% identity with SEQ ID NO: 2. In another particular embodiment of the method of the invention the target region or the amplification reaction product consists of SEQ ID NO: 2.

The expression "fragment thereof", as used herein, refers to a fragment of the nucleotide sequence according to SEQ ID NO: 1, wherein at least 1 nucleotide from the 5' and/or at least one nucleotide from the 3' has been deleted. In a particular embodiment the fragment thereof consists of a nucleotide sequence of at least 20, at least 21, at least 22, at least 23, at least 24, at least 25 nucleotides wherein said nucleotide sequence is comprised in the nucleotide sequence according to SEQ ID NO: 1.

The terms "identity", "identical" or "percent identity" in the context of two or more amino acid or nucleotide sequences, refer to two or more sequences or subsequences that are the same or have a specified percentage of nucleotide residues that are the same, when compared and aligned (introducing gaps, if necessary) for maximum correspondence, not considering any conservative substitutions as part of the sequence identity. The percent identity can be measured using sequence comparison software or algorithms or by visual inspection. Various algorithms and software are known in the art that can be used to obtain alignments of amino acid or nucleotide sequences. Publicly available software programs can be used to align sequences. Appropriate parameters for maximal alignment by particular alignment software can be determined by one skilled in the art. In certain embodiments, the default parameters of the alignment software are used. In certain embodiments, the percentage identity "X" of a first nucleotide sequence to a second nucleotide sequence is calculated as 100 x (Y/Z), where Y is the number of nucleotide residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

For instance, 95% identical to a reference sequence according to the present invention, the parameters are set such that the percentage of identity is calculated over the full length of the reference nucleotide sequence and that gaps in homology of up to 5% of the total number of nucleotides in the reference sequence are allowed.

The amplification reaction which amplifies the target region comprises a forward primer and a reverse primer which are specific and capable of amplifying said region or fragment thereof. The term "primer", as used herein, refers to an oligonucleotide that is capable of acting as an initiation point for the 5' to 3' synthesis of a primer extension product that is complementary to a nucleic acid strand. The primer extension product is synthesized in the presence of appropriate nucleotides and a polymerization agent, such as a DNA polymerase, in an appropriate buffer and at an appropriate temperature. The primers may comprise only natural nucleotide residues or at least one or more nucleotide analogues, e.g., chemically modified analogues, PNAs, etc.

Examples of modified nucleotides that can be used in the present invention include, but are not limited to, nucleotides having at position 2' of the sugar a substituent selected from the fluoro, hydroxyl, amino, azido, alkyl, alkoxy, alkoxyalkyl, methyl, ethyl, propyl, butyl group or a functionalized alkyl group such as ethylamino, propylamino and butylamino. Alternatively, the alkoxy group is methoxy, ethoxy, propoxy or a functionalized alkoxy group according to the formula O(CH2)q-R, where q is 2 to 4 and R is an amino, methoxy or ethoxy group. Suitable alkoxyalkyl groups are methoxyethyl and ethoxyethyl. Oligonucleotides in which different nucleotides contain different modifications at position 2' are also part of the invention. Alternatively or additionally, the oligonucleotides of the invention can contain modified bonds such as phosphodiester-, phosphotriester-, phosphorothioate-, phosphorodithioate-, phosphoroselenoate-, phosphorodiselenoate-, phosphoroanilothioate-, phosphoramidate-, methylphosphonate-, boranephosphonatetype bonds as well as combinations thereof, or they are peptide nucleic acids (PNAs), in which the different nucleotides are bound by amide bonds.

Normally two types of primers are used, a "forward primer" which anneals with the antisense DNA strand and initiates the synthesis of positive strand of the gene into 5' to 3' direction and a "reverse primer" which anneals with the sense strand and initiates the synthesis of the complementary strand of the coding strand, which is negative strand of the gene into 5' to 3' direction. The 5' ends of both primers bind to the 3' ends of each DNA strand. The primers are, in most cases, short oligonucleotides. In a particular embodiment of the method I, method II or method III of the method of the invention the primers comprise 18 to 30 nucleotides, preferably 19, 20, 21, 22, 23, 24, 25, 26, 27, 28 or 29 nucleotides, more preferably 20, 21 or 22 nucleotides. In another particular embodiment the forward primer consists of a sequence selected from the group consisting of SEQ ID NO: 3 and SEQ ID NO: 4. In yet another particular embodiment the reverse primer consists of a sequence selected from the group consisting of SEQ ID NO: 5 and SEQ ID NO: 6.

The expression "specific and capable of amplifying said target region or fragment thereof", as used in the present description, means that the primers are designed to be complementary to the target DNA sequence to be amplified (i.e., refers to the base pairing that allows the formation of a duplex between the primer and its complementary sequence in a DNA molecule). Two single-stranded DNA molecules are said to be substantially complementary when the nucleotides of one strand, optimally aligned and compared and with appropriate nucleotide insertions or deletions, pair with about 60% of the other strand, at least 70%, at least 80%, at least 85%, usually at least about 90% to about 95%, and even about 98% to about 100%. "Specific" means that the primers are capable of hybridizing to and amplify the nucleic acid target region, which may be part of the genome of a *Bacteroidetes,* particularly the genome *Bacteroides* spp., more particularly of P. *dorei,* but they are not capable of hybridizing and amplifying other sequences different, thus allowing a specific detection of said nucleic acid sequence. In a particular embodiment of the method I, method II and method III of the method of the invention, the primers are able to amplify the 16S RNA gene of the genome of a *Bacteroidetes,* preferably a *Bacteroides spp.,* more preferably of *P*. *dorei.*

In a preferred embodiment, the expression "specific and capable of amplifying said target region or fragment thereof", as used in the present description, refers to the fact that one of the primers used in the amplification reaction comprises a sequence that is identical to a fragment of SEQ ID NO. 2 and the other primer comprises a sequence that is identical to a reverse and complementary fragment to SEQ ID NO. 2, and that the two primers together, in an amplification reaction, hybridize with the single DNA chains of SEQ ID NO: 2 and allow for the amplification of SEQ ID NO: 1 or a fragment thereof.

The amplification reaction may, in addition, comprise components necessary for the detection or quantification of the amplification products or amplicons or for the determination of the nucleic acid sequence of the amplicon. Thus, while the amplification reaction in method I of the method of the invention is performed in the presence of an oligonucleotide probe, method II of the method of the invention comprises a hybridization reaction step (step ii) of the amplification reaction product performed with an oligonucleotide probe and its complementary strand from the amplification reaction product bind non-covalently to form a stable double-stranded polynucleotide, while method III does not comprise oligonucleotide probes as the amplicon nucleotide sequence is determined. In both I and II cases the oligonucleotide probe comprises at least one label capable of emitting a detectable signal and the oligonucleotide probe sequence hybridizes to the nucleotide sequence according to SEQ ID NO: 1 or a fragment thereof. In a particular embodiment of method I or method II of the method of the invention, the oligonucleotide probe comprises a sequence according to SEQ ID NO: 1, preferably consists of a sequence according to SEQ ID NO: 1.

As used herein, the term 'probe' refers to a molecule used in an amplification reaction or in a hybridization reaction, such as for quantitative or qPCR analysis, as well as for end-point analysis. Such probes can be used to detect the presence of specific nucleotide sequences present in the target region both during the amplification reaction and in end-point analysis, using as in hydribidization reactions. In a particular embodiment of method I or method II of the method of the invention, the oligonucleotide probe consists of or has between 18 to 30 nucleotides, preferably between 20 to 26 nucleotides, more preferably 20 nucleotides, 21 nucleotides, 22 nucleotides, 23 nucleotides, 24 nucleotides or 25 nucleotides.

Such oligonucleotide probes may be modified with a label so that the appearance or the detection of amplification reaction products with a nucleotide sequence reversed and complementary to the probe is detected by the appearance of a detectable signal. The term "label", as used herein, refers to any atom or molecule that can be used to provide a detectable effect, and that can be attached to a nucleic acid. Labels include, but are not limited to, dyes; radiolabels such as 32P; binding moieties such as biotin; haptens such as digoxygenin; luminogenic, phosphorescent or fluorogenic moieties; mass tags; and fluorochromes alone or in combination with quenchers that can suppress or shift emission spectra by fluorescence resonance energy transfer (FRET). Said labels may provide signals detectable by fluorescence, radioactivity, colorimetry, gravimetry, X-ray diffraction or absorption, magnetism, enzymatic activity, characteristics of mass or behavior affected by mass (e.g., MALDI time-of-flight mass spectrometry), and the like, preferably by fluorescence. A label may be a charged moiety (positive or negative charge) or alternatively, may be charge neutral. Labels can include or consist of nucleic acid or protein sequence, so long as the sequence comprising the label is detectable. In a particular embodiment of the method I or method II of the invention the probe comprises at least one label. In yet another particular embodiment of the method I or method II of the method of the invention the label is selected from the group consisting of: biotin, digoxigenin, radioactively-labelled deoxynucleoside triphosphate (dNTP), 4-acetamido-4'-isothiocyanatostilbene-2,2' disulfonic acid, acridine, acridine orange, acridine yellow, acridine red, acridine isothiocyanate, cyanosine (Cy), Cy3, Cy5, Cy5.5, Cy7, 4',6-diaminidino-2-phenylindole (DAPI); 5',5"-dibromopyrogallol-sulfonephthalein (Bromopyrogallol Red), eosin, eosin isothiocyanate, erythrosin B, erythrosin isothiocyanate, ethidium, fluorescein, 5-carboxyfluorescein (FAM), 5-(4,6-dichlorotriazin-2-yl)aminofluorescein (DTAF), pyrene, pyrene butyrate, succinimidyl 1-pyrene butyrate, Reactive Red 4 (Cibacron^{™} Brilliant Red 3B-A), rhodamine, 6-carboxy-X-rhodamine (ROX), 6-carboxyrhodamine (R6G), tetramethyl rhodamine (TAMRA), tetramethyl rhodamine isothiocyanate (TRITC), riboflavin, rosolic acid, terbium chelate, xanthene, Alexa Fluor 350, Alexa Fluor 430, Alexa Fluor 488, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, Alexa Fluor 750, Pacific Blue, Pacific Orange, Cascade Blue, Cascade Yellow, Quantum Dot dyes (Quantum Dot Corporation), Dylight 800, Dylight 680, Dylight 649, Dylight 633, Dylight 549, Dylight 488, Dylight 405 and any combination thereof.

The interaction between two labels may produce a detectable effect. The interaction is not limited to any particular nature of interaction. The interaction of the labels may be via direct contact, e.g., a covalent or non-covalent contact between two moieties (e.g., a protein-protein contact, or collisional energy transfer between proximal moieties); it may comprise resonance energy transfer (e.g., between one or more dyes, or between a dye and a quencher moieties); it may comprise a diffusion effect, e.g., wherein the product from a reaction occurring at the site of one label diffuses to the site of another label to create a detectable effect.

In a particular embodiment of the method I or method II of the method of the invention the oligonucleotide probe may contain more than one label. In a particular embodiment of method I or method II of the method of the invention, the oligonucleotide probe comprises two labels, wherein one label is a fluorophore located in the terminal 5' or 3' and the other label is a quencher molecule in the opposite terminal.

The rationale for using two labels on the oligonucleotide probe, a donor label and an acceptor label, is based on the use of Forster resonance energy transfer, which is an energy transfer mechanism between fluorochromes, where the presence of an energy acceptor near an energy donor blocks the fluorescence emission of the energy donor. Various types of probes can be used, such as, without limitation, TaqMan probes and Molecular Beacons. TaqMan probes refer to probes that have a fluorophore at one end and a molecule at the other end that blocks its fluorescence emission (called a "quencher"). This type of probe hybridizes specifically to the central part of the PCR product to be obtained. Thus, when the amplification reaction is performed, the probe hybridizes to the target region but, due to the proximity of the fluorophore to the quencher, no fluorescence is emitted. When the polymerase encounters the probe, it hydrolyses it via its 5'-3' exonuclease activity, resulting in the separation of the quencher from the fluorophore and thus the emission of fluorescence, which is directly related to the amount of amplicon produced.

In a particular embodiment of the method I or method II of the method of the invention the oligonucleotide probe comprises a fluorophore selected from the group consisting of:
- 3',6'-dihydroxy-1-oxospiro[2-benzofuran-3,9'-xanthene]-5-carboxylic acid (6-FAM);
- [2',4,4',5',7,7'-hexachloro-6-[6-[2-cyanoethoxy-[di(propan-2-yl)amino]phosphanyl]oxyhexylcarbamoyl]-6'-(2,2-dimethylpropanoyloxy)-3-oxospiro[2-benzofuran-1,9'-xanthene]-3'-yl] 2,2-dimethylpropanoate (HEX);
- (2Z)-2-[(3,6-dimethyl-2-phenylpyrimidin-3-ium-4-yl)methylidene]-1-ethylquinoline, chloride (Cy3, Cy5);
- (3*R*,4*S*,5*S*,6*R*,7*R*,9*R*,11*S*,12*R*,13*S*,14*R*)-6-[(2*S*,3*R*,4*S*,6*R*)-4-(dimethylamino)-3-hydroxy-6-methyloxan-2-yl]oxy-14-ethyl-7,12,13-trihydroxy-4-[(2*R*,4*R*,5*S*,6*S*)-5-hydroxy-4-methoxy-4,6-dimethyloxan-2-yl]oxy-10-(2-methoxyethoxymethoxyamino)-3,5,7,9,11,13-hexamethyl-oxacyclotetradecan-2-one (ROX);
- 5-chlorosulfonyl-2-(3-oxa-23-aza-9-azoniaheptacyclo[17.7.1.1^{5,9}.0^{2,17}.0^{4,15}.0^{23,27}.0^{13,28}]octacosa-1(27),2(17),4,9(28),13,15,18-heptaen-16-yl)benzenesulfonate (Texas Red);
- 2-(7-ethyl-3,3,8,8,10-pentamethyl-7-aza-21-azoniahexacyclo[15.7.1.0^{2,15}.0^{4,13}.0^{6,11}.0^{21,25}]pentacosa-1,4(13),5,11,14,16,18,21(25)-octaen-14-yl)-*N*-methyl-*N*-(4-oxopentyl)benzamide (ATTON 647N);
- (2,5-dioxopyrrolidin-1-yl) 4',5'-dichloro-3',6'-dihydroxy-2',7'-dimethoxy-1-oxospiro[2-benzofuran-3,9'-xanthene]-5-carboxylate (6-JOE);
- (2S,4*R*)-*N*-[(1*S*)-2-methyl-1-[(2*R*,3*R*,4*S*,5*R*,6*R*)-3,4,5-trihydroxy-6-methylsulfanyloxan-2-yl]propyl]-4-propylpiperidine-2-carboxamide (VIC); and
- (tetrachlorofluorescein): 4,5,6,7-tetrachloro-3',6'-dihydroxyspiro[2-benzofuran-3,9'-xanthene]-1-one (TET).

In a particular embodiment of the method of the invention the oligonucleotide probe comprises a quencher molecule selected from the group consisting of:
- *N*-[2-[[(*E*)-3-[1-[(2*R*,4*S*,5*R*)-5-[[bis(4-methoxyphenyl)-phenylmethoxy]methyl]-4-[2-cyanoethoxy-[di(propan-2-yl)amino]phosphanyl]oxyoxolan-2-yl]-2,4-dioxopyrimidin-5-yl]prop-2-enoyl]amino]ethyl]-6-[[7-[[2,5-dimethoxy-4-[(4-nitrophenyl)diazenyl]phenyl]diazenyl]-1-azatricyclo[7.3.1.0^{5,13}]trideca-5(13),6,8-trien-6-yl]oxy]hexanamide (BBQ650);
- 2-[3-(dimethylamino)-6-dimethylazaniumylidenexanthen-9-yl]benzoate (TAMRA);
- (2S)-2-[4-(3,4-dimethylphenyl)-2-methylquinolin-3-yl]-2-[(2-methylpropan-2-yl)oxy]acetic acid (TQ2);
- 5-phenylsulfanylquinazoline-2,4-diamine (TQ3); and
- 2,5-Bis(2-methyl-2-propanyl)-1,4-benzenediol (BHQ-1 and 2).

In a particular embodiment, the methods of the invention comprise a step of administering to the subject from whom the target region has been detected in the sample, a treatment for ulcerative colitis.

The term "treatment", as used herein, refers to any process, action, application, or the like, wherein a subject or patient, including a human being, with a specific health condition or disorder is provided medical aid with the objective of improving the subject's condition, directly or indirectly, or slowing the progression of a condition or disorder in the subject, or ameliorating at least one symptom of the condition or disorder under treatment. In a preferred embodiment, a treatment or therapy refers to the administration at a specific regimen or dose for a given period of time, of a specific active agent, or a combination of active agents, comprised in a medicament or set of medicaments.

Non-limiting examples of treatments for ulcerative colitis comprise surgery or drug therapy, including anti-inflammatory drugs (5-aminosalicylates such as sulfasalazine, mesalamine, balsalazide and olsalazine; corticosteroids such as prednisone and budesonide), immune system suppressors (azathioprine, mercaptopurine, cyclosporine, tofacitinib, etc.), biologics (infliximab, adalimumab, golimumab, vedolizumab, ustekinumab, etc.), anti-diarrheal medications (loperamide, etc.), pain relievers (acetaminophen, etc.), antispasmodics, or iron supplements.

### Oligonucleotides of the invention

The method of the invention makes use of oligonucleotides in order for amplification reactions to be carried out as to identify bacteria which are associated with subjects suffering from UC. Such oligonucleotides are used as primers and probes, allowing the method of the invention to be carried out in its several forms.

Therefore, a further aspect of the present invention relates to an oligonucleotide, from here onwards the oligonucleotide of the invention, comprising a nucleotide sequence according to SEQ ID NO: 4 or SEQ ID NO: 6 or a combination thereof; preferably consisting of a nucleotide sequence according to SEQ ID NO: 4 or SEQ ID NO: 6 or a combination thereof.

Another aspect relates to an oligonucleotide probe, form here onwards the probe of the invention, comprising a nucleotide sequence according to SEQ ID NO: 1; preferably consisting of a nucleotide sequence according to SEQ ID NO: 1.

All terms and definitions previously described are equally applicable to the present aspects.

The term "oligonucleotide", as used herein, refers to a single stranded or double stranded sequence of nucleic acids, more preferably of ribonucleic acid (RNA) or deoxyribonucleic acid (DNA) or mimetics thereof; said nucleic acids may be sense or antisense. In a particular embodiment of the method of the invention invention, an oligonucleotide includes 5 to 50 nucleotides, more preferably 9 to 30 nucleotides and even more preferably from 13 to 25 nucleotides.

In a particular embodiment the probe of the invention comprises one or more labels. In a more particular embodiment of the probe of the invention the label is selected from the group consisting of: biotin, digoxigenin, radioactively-labelled deoxynucleoside triphosphate (dNTP), 4-acetamido-4'-isothiocyanatostilbene-2,2' disulfonic acid, acridine, acridine orange, acridine yellow, acridine red, acridine isothiocyanate, cyanosine (Cy), Cy3, Cy5, Cy5.5, Cy7, 4',6-diaminidino-2-phenylindole (DAPI); 5',5"-dibromopyrogallol-sulfonephthalein (Bromopyrogallol Red), eosin, eosin isothiocyanate, erythrosin B, erythrosin isothiocyanate, ethidium, fluorescein, 5-carboxyfluorescein (FAM), 5-(4,6-dichlorotriazin-2-yl)aminofluorescein (DTAF), pyrene, pyrene butyrate, succinimidyl 1-pyrene butyrate, Reactive Red 4 (Cibacron^{™} Brilliant Red 3B-A), rhodamine, 6-carboxy-X-rhodamine (ROX), 6-carboxyrhodamine (R6G), tetramethyl rhodamine (TAMRA), tetramethyl rhodamine isothiocyanate (TRITC), riboflavin, rosolic acid, terbium chelate, xanthene, Alexa Fluor 350, Alexa Fluor 430, Alexa Fluor 488, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, Alexa Fluor 750, Pacific Blue, Pacific Orange, Cascade Blue, Cascade Yellow, Quantum Dot dyes (Quantum Dot Corporation), Dylight 800, Dylight 680, Dylight 649, Dylight 633, Dylight 549, Dylight 488, Dylight 405 and any combination thereof.

In a particular embodiment of the probe of the invention, the probe may contain more than one label. In a particular embodiment the probe of the invention comprises two labels, wherein one label is a fluorophore located in the terminal 5' or 3' and the other label is a quencher molecule in the opposite terminal.

In a particular embodiment, the probe of the invention comprises a fluorophore selected from the group consisting of:
- 3',6'-dihydroxy-1-oxospiro[2-benzofuran-3,9'-xanthene]-5-carboxylic acid (6-FAM);
- [2',4,4',5',7,7'-hexachloro-6-[6-[2-cyanoethoxy-[di(propan-2-yl)amino]phosphanyl]oxyhexylcarbamoyl]-6'-(2,2-dimethylpropanoyloxy)-3-oxospiro[2-benzofuran-1,9'-xanthene]-3'-yl] 2,2-dimethylpropanoate (HEX);
- (2Z)-2-[(3,6-dimethyl-2-phenylpyrimidin-3-ium-4-yl)methylidene]-1-ethylquinoline, chloride (Cy3, Cy5);
- (3*R*,4*S*,5*S*,6*R*,7*R*,9*R*,11*S*,12*R*,13*S*,14*R*)-6-[(2*S*,3*R*,4*S*,6*R*)-4-(dimethylamino)-3-hydroxy-6-methyloxan-2-yl]oxy-14-ethyl-7,12,13-trihydroxy-4-[(2*R*,4*R*,5*S*,6*S*)-5-hydroxy-4-methoxy-4,6-dimethyloxan-2-yl]oxy-10-(2-methoxyethoxymethoxyamino)-3,5,7,9,11,13-hexamethyl-oxacyclotetradecan-2-one (ROX);
- 5-chlorosulfonyl-2-(3-oxa-23-aza-9-azoniaheptacyclo[17.7.1.1^{5,9}.0^{2,17}.0^{4,15}.0^{23,27}.0^{13,28}]octacosa-1(27),2(17),4,9(28),13,15,18-heptaen-16-yl)benzenesulfonate (Texas Red);
- 2-(7-ethyl-3,3,8,8,10-pentamethyl-7-aza-21-azoniahexacyclo[15.7.1.0^{2,15}.0^{4,13}.0^{6,11}.0^{21,25}]pentacosa-1,4(13),5,11,14,16,18,21(25)-octaen-14-yl)-*N*-methyl-*N*-(4-oxopentyl)benzamide (ATTON 647N);
- (2,5-dioxopyrrolidin-1-yl) 4',5'-dichloro-3',6'-dihydroxy-2',7'-dimethoxy-1-oxospiro[2-benzofuran-3,9'-xanthene]-5-carboxylate (6-JOE);
- (2*S*,4*R*)-*N*-[(1*S*)-2-methyl-1-[(2*R*,3*R*,4*S*,5*R*,6*R*)-3,4,5-trihydroxy-6-methylsulfanyloxan-2-yl]propyl]-4-propylpiperidine-2-carboxamide (VIC); and
- (tetrachlorofluorescein): 4,5,6,7-tetrachloro-3',6'-dihydroxyspiro[2-benzofuran-3,9'-xanthene]-1-one (TET).

In another particular embodiment the probe of the invention comprises a quencher molecule selected from the group consisting of:
- *N*-[2-[[(*E*)-3-[1-[(2*R*,4*S*,5*R*)-5-[[bis(4-methoxyphenyl)-phenylmethoxy]methyl]-4-[2-cyanoethoxy-[di(propan-2-yl)amino]phosphanyl]oxyoxolan-2-yl]-2,4-dioxopyrimidin-5-yl]prop-2-enoyl]amino]ethyl]-6-[[7-[[2,5-dimethoxy-4-[(4-nitrophenyl)diazenyl]phenyl]diazenyl]-1-azatricyclo[7.3.1.0^{5,13}]trideca-5(13),6,8-trien-6-yl]oxy]hexanamide (BBQ650);
- 2-[3-(dimethylamino)-6-dimethylazaniumylidenexanthen-9-yl]benzoate (TAMRA);
- (2S)-2-[4-(3,4-dimethylphenyl)-2-methylquinolin-3-yl]-2-[(2-methylpropan-2-yl)oxy]acetic acid (TQ2);
- 5-phenylsulfanylquinazoline-2,4-diamine (TQ3); and
- 2,5-Bis(2-methyl-2-propanyl)-*1*,4-benzenediol (BHQ-1 and 2).

### Kit of the invention

The present invention can be adapted to be performed by products and or materials provided in a kit. Therefore, another aspect of the present invention relates to a kit, from here onwards the kit of the invention, comprising the probe of the invention and/or the oligonucleotide of the invention.

All terms and definitions previously described are equally applicable to the present aspects.

In the context of the present invention, "kit" is understood as a product of the different reagents for performing the methods described in the present invention, both in those cases in which the amplification reaction is performed with primers and a probe and in the cases in which the amplification reaction is performed first with the primers and subsequently the hybridization reaction is performed, in which the different reagents are packaged together to allow for transport and storage. Nevertheless, if the kits defined in the present invention do not comprise the reagents necessary for putting the methods of the invention into practice, such reagents are commercially available and can be found as part of a kit. Suitable materials for packaging the components of the kit include, without being limited to, glass, plastic (polyethylene, polypropylene, polycarbonate and the like), bottles, vials, paper, sachets and the like. Kits can additionally contain instructions for using the different components in the kit. Said instructions can be in printed format or in an electronic device capable of storing instructions such that they can be read by a person, such as electronic storage media (magnetic discs, tapes and the like), optical means (CD-ROM, DVD, USB) and the like. The media can additionally or alternatively contain Internet addresses where said instructions are provided.

In a particular embodiment of the kit of the invention, the kit further comprises at least one forward primer and one reversed primer, wherein the forward primer is SEQ ID NO: 3 and the reverse primer is SEQ ID NO: 5.

### Uses of the invention

The present invention discloses several aspects which find use in the diagnosis of UC in a subject or for the detection of *Bacteriodetes* bacteria associated with UC. As such, another aspect of the present invention relates to the use of the oligonucleotide of the invention or the probe of the invention or the kit of the invention, from here onwards the diagnostic use of the invention, for the diagnosis of ulcerative colitis in a subject.

Another aspect of the present invention relates to the use of the oligonucleotide of the invention or the probe of the invention or the kit of the invention, from here onwards the detection use of the invention, for the detection and/or identification of bacteria associated with ulcerative colitis.

All terms and definitions previously described are equally applicable to the present aspects.

In a particular embodiment of the diagnostic use of the invention the diagnosis is performed by the method of the invention.

The present invention is further defined by the following up aspects:
1. An *in vitro* method for the diagnosis of ulcerative colitis in a subject, wherein the method comprises detecting a nucleic acid target region in a sample of feces from the subject, wherein said region comprises the sequence according to SEQ ID NO: 1, the sequence complementary to SEQ ID NO: 1, or a fragment thereof, wherein the presence of the target region is indicative of the subject having ulcerative colitis.
2. An *in vitro* method for the diagnosis of ulcerative colitis in a subject wherein the method comprises detecting a nucleic acid sequence in a sample of feces from the subject, wherein said region comprises the sequence according to SEQ ID NO: 9, the sequence complementary to SEQ ID NO: 9, or a fragment thereof, preferably the sequence according to SEQ ID NO: 2, the sequence complementary to SEQ ID NO: 2, or a fragment thereof, and wherein the presence of the nucleic acid sequence is indicative of the subject having ulcerative colitis.
3. The *in vitro* method according to aspect 1 or 2, wherein the method comprises the steps of:
   (i) submitting the nucleic acids obtained from the sample of the subject to an amplification reaction of the nucleic acid target region obtaining an amplification reaction product wherein the amplification reaction product comprises the nucleotide sequence according to SEQ ID NO: 1 or a fragment thereof and wherein the amplification reaction comprises a forward primer, a reverse primer and an oligonucleotide probe, wherein the forward primer and the reverse primer are specific and capable of amplifying said amplification reaction product, the oligonucleotide probe comprises at least one label capable of emitting a detectable signal and the oligonucleotide probe sequence hybridizes to the nucleotide sequence according to SEQ ID NO: 1 or fragment thereof; and
   (ii) detecting the signal emitted by the label during the amplification reaction in step (i),
   wherein the detection of the signal emitted by the label is indicative of the subject having ulcerative colitis.
4. The *in vitro* method according to aspect 1 or 2, wherein the method comprises the steps of:
   (i) submitting the nucleic acids obtained from the sample of the subject to an amplification reaction of the nucleic acid target region obtaining an amplification reaction product wherein the amplification reaction product comprises the nucleotide sequence according to SEQ ID NO: 1 or a fragment thereof and wherein the amplification reaction comprises a forward primer and a reverse primer wherein the forward primer and the reverse primer are specific and capable of amplifying said amplification reaction product;
   (ii) performing an hybridization reaction of the amplification reaction product with an oligonucleotide probe that hybridizes to the nucleotide sequence according to SEQ ID NO: 1 or a fragment thereof wherein said oligonucleotide probe comprises at least one label capable of emitting a detectable signal, and
   (iii) detecting the signal emitted by the label after the hybridization reaction of step (ii),
   wherein the detection of the signal emitted by the label is indicative of the subject having ulcerative colitis.
5. The method according to aspect 1 or 2, wherein the method comprises the steps of:
   (i) submitting the nucleic acids obtained from the sample of the subject to an amplification reaction of the nucleic acid target region obtaining an amplification reaction product wherein the amplification reaction product comprises the nucleotide sequence according to SEQ ID NO: 1, or a fragment thereof, or SEQ ID NO: 2 or a fragment thereof, and wherein the amplification reaction comprises a forward primer, a reverse primer, wherein the forward primer and the reverse primer are specific and capable of amplifying said amplification reaction product; and
   (ii) sequencing the amplification reaction product,
   wherein the presence in the amplification reaction product of a nucleic acid sequence which comprises or consists of SEQ ID NO: 1 is indicative of the subject having ulcerative colitis.
6. The method according to aspect 5 wherein the amplification reaction further comprises an oligonucleotide probe.
7. The method according to any one of aspects 1 to 6, wherein the target region belongs to *Bacteroidetes* bacteria which comprises in its genome the sequence according to SEQ ID NO: 1 or SEQ ID NO: 2.
8. The method according to any one of aspects 1 to 7, wherein the nucleic acid target region further comprises the nucleic acid sequence according to SEQ ID NO: 18.
9. The method according to any one of aspects 1 to 8, wherein the nucleic acid target region further comprises the nucleic acid sequence according to SEQ ID NO: 4.
10. The method according to any one of aspects 1 to 9, wherein the target region consists of SEQ ID NO: 1.
11. The method according to any one of aspects 3 to 10, wherein the amplification reaction is a real time quantitative polymerase chain reaction (PCR) or a real time semi-quantitative polymerase chain reaction (PCR).
12. The method according to any one of aspects 3 to 11, wherein the primers comprise 18 to 30 nucleotides, preferably 20 or 22 nucleotides.
13. The method according to any one of aspects 3 to 12, wherein the forward primer consists of a sequence selected from the group consisting of SEQ ID NO: 3 and SEQ ID NO: 4.
14. The method according to any one of aspects 3 to 13, wherein the reverse primer consists of a sequence selected from the group consisting of SEQ ID NO: 5 and SEQ ID NO: 6.
15. The method according to any one of aspects 3 to 14, wherein the oligonucleotide probe consists of a sequence according to SEQ ID NO: 1.
16. The method according to any one of aspects 3 to 15 wherein the amplification reaction product comprises a nucleotide sequence with at least 80% identity with a nucleotide sequence according to SEQ ID NO: 2.
17. The method according to any one of aspects 3 to 16 wherein the reaction product comprises the 16S RNA gene of the genome of a *Bacteroidetes,* preferably a *Bacteroides spp.,* more preferably of *P*. *dorei.*
18. The method according to any one of aspects 3 to 17, wherein the label is selected from the group consisting of: biotin, digoxigenin, radioactively-labelled deoxynucleoside triphosphate (dNTP), 4-acetamido-4'-isothiocyanatostilbene-2,2' disulfonic acid, acridine, acridine orange, acridine yellow, acridine red, acridine isothiocyanate, cyanosine (Cy), Cy3, Cy5, Cy5.5, Cy7, 4',6-diaminidino-2-phenylindole (DAPI); 5',5"-dibromopyrogallol-sulfonephthalein (Bromopyrogallol Red), eosin, eosin isothiocyanate, erythrosin B, erythrosin isothiocyanate, ethidium, fluorescein, 5-carboxyfluorescein (FAM), 5-(4,6-dichlorotriazin-2-yl)aminofluorescein (DTAF), pyrene, pyrene butyrate, succinimidyl 1-pyrene butyrate, Reactive Red 4 (Cibacron^{™} Brilliant Red 3B-A), rhodamine, 6-carboxy-X-rhodamine (ROX), 6-carboxyrhodamine (R6G), tetramethyl rhodamine (TAMRA), tetramethyl rhodamine isothiocyanate (TRITC), riboflavin, rosolic acid, terbium chelate, xanthene, Alexa Fluor 350, Alexa Fluor 430, Alexa Fluor 488, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, Alexa Fluor 750, Pacific Blue, Pacific Orange, Cascade Blue, Cascade Yellow, Quantum Dot dyes (Quantum Dot Corporation), Dylight 800, Dylight 680, Dylight 649, Dylight 633, Dylight 549, Dylight 488, Dylight 405 and any combination thereof.
19. The method according to any one of aspects 3 to 18, wherein the oligonucleotide probe comprises two labels, wherein one label is a fluorophore located in the terminal 5' or 3' and the other label is a quencher molecule in the opposite terminal.
20. The method according to aspect 19, wherein the fluorophore is selected from the group consisting of:
   - 3',6'-dihydroxy-1-oxospiro[2-benzofuran-3,9'-xanthene]-5-carboxylic acid (6-FAM);
   - [2',4,4',5',7,7'-hexachloro-6-[6-[2-cyanoethoxy-[di(propan-2-yl)amino]phosphanyl]oxyhexylcarbamoyl]-6'-(2,2-dimethylpropanoyloxy)-3-oxospiro[2-benzofuran-1,9'-xanthene]-3'-yl] 2,2-dimethylpropanoate (HEX);
   - (2*Z*)-2-[(3,6-dimethyl-2-phenylpyrimidin-3-ium-4-yl)methylidene]-1-ethylquinoline, chloride (Cy3, Cy5);
   - (3*R*,4*S*,5*S*,6*R*,7*R*,9*R*,11*S*,12*R*,13*S*,14*R*)-6-[(2*S*,3*R*,4*S*,6*R*)-4-(dimethylamino)-3-hydroxy-6-methyloxan-2-yl]oxy-14-ethyl-7,12,13-trihydroxy-4-[(2*R*,4*R*,5*S*,6*S*)-5-hydroxy-4-methoxy-4,6-dimethyloxan-2-yl]oxy-10-(2-methoxyethoxymethoxyamino)-3,5,7,9,11,13-hexamethyl-oxacyclotetradecan-2-one (ROX);
   - 5-chlorosulfonyl-2-(3-oxa-23-aza-9-azoniaheptacyclo[17.7.1.1^{5,9}.0^{2,17}.0^{4,15}.0^{23,27}.0^{13,28}]octacosa-1(27),2(17),4,9(28),13,15,18-heptaen-16-yl)benzenesulfonate (Texas Red);
   - 2-(7-ethyl-3,3,8,8,10-pentamethyl-7-aza-21-azoniahexacyclo[15.7.1.0^{2,15}.0^{4,13}.0^{6,11}.0^{21,25}]pentacosa-1,4(13),5,11,14,16,18,21(25)-octaen-14-yl)-*N*-methyl-*N*-(4-oxopentyl)benzamide (ATTON 647N);
   - (2,5-dioxopyrrolidin-1-yl) 4',5'-dichloro-3',6'-dihydroxy-2',7'-dimethoxy-1-oxospiro[2-benzofuran-3,9'-xanthene]-5-carboxylate (6-JOE);
   - (2*S*,4*R*)-*N*-[(1*S*)-2-methyl-1-[(2*R*,3*R*,4*S*,5*R*,6*R*)-3,4,5-trihydroxy-6-methylsulfanyloxan-2-yl]propyl]-4-propylpiperidine-2-carboxamide (VIC); and
   - (tetrachlorofluorescein): 4,5,6,7-tetrachloro-3',6'-dihydroxyspiro[2-benzofuran-3,9'-xanthene]-1-one (TET).
21. The method according to aspect 19 or aspect 20, wherein the quencher molecule is selected from the group consisting of:
   - *N*-[2-[[(*E*)-3-[1-[(2*R*,4*S*,5*R*)-5-[[bis(4-methoxyphenyl)-phenylmethoxy]methyl]-4-[2-cyanoethoxy-[di(propan-2-yl)amino]phosphanyl]oxyoxolan-2-yl]-2,4-dioxopyrimidin-5-yl]prop-2-enoyl]amino]ethyl]-6-[[7-[[2,5-dimethoxy-4-[(4-nitrophenyl)diazenyl]phenyl]diazenyl]-1-azatricyclo[7.3.1.0^{5,13}]trideca-5(13),6,8-trien-6-yl]oxy]hexanamide (BBQ650);
   - 2-[3-(dimethylamino)-6-dimethylazaniumylidenexanthen-9-yl]benzoate (TAMRA);
   - (2S)-2-[4-(3,4-dimethylphenyl)-2-methylquinolin-3-yl]-2-[(2-methylpropan-2-yl)oxy]acetic acid (TQ2);
   - 5-phenylsulfanylquinazoline-2,4-diamine (TQ3); and
   - 2,5-Bis(2-methyl-2-propanyl)-1,4-benzenediol (BHQ-1 and 2).
22. The method according to any one of aspects 1 to 21, wherein the subject is a mammal, preferably a human.
23. An oligonucleotide consisting of a nucleotide sequence according to SEQ ID NO: 4 or SEQ ID NO: 6 or a combination thereof.
24. An oligonucleotide probe consisting of a nucleotide sequence according to SEQ ID NO: 1.
25. The oligonucleotide probe according to aspect 24, comprising at least one label selected from the group consisting of: biotin, digoxigenin, radioactively-labelled deoxynucleoside triphosphate (dNTP), 4-acetamido-4'-isothiocyanatostilbene-2,2' disulfonic acid, acridine, acridine orange, acridine yellow, acridine red, acridine isothiocyanate, cyanosine (Cy), Cy3, Cy5, Cy5.5, Cy7, 4',6-diaminidino-2-phenylindole (DAPI); 5',5"-dibromopyrogallol-sulfonephthalein (Bromopyrogallol Red), eosin, eosin isothiocyanate, erythrosin B, erythrosin isothiocyanate, ethidium, fluorescein, 5-carboxyfluorescein (FAM), 5-(4,6-dichlorotriazin-2-yl)aminofluorescein (DTAF), pyrene, pyrene butyrate, succinimidyl 1-pyrene butyrate, Reactive Red 4 (Cibacron^{™} Brilliant Red 3B-A), rhodamine, 6-carboxy-X-rhodamine (ROX), 6-carboxyrhodamine (R6G), tetramethyl rhodamine (TAMRA), tetramethyl rhodamine isothiocyanate (TRITC), riboflavin, rosolic acid, terbium chelate, xanthene, Alexa Fluor 350, Alexa Fluor 430, Alexa Fluor 488, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, Alexa Fluor 750, Pacific Blue, Pacific Orange, Cascade Blue, Cascade Yellow, Quantum Dot dyes (Quantum Dot Corporation), Dylight 800, Dylight 680, Dylight 649, Dylight 633, Dylight 549, Dylight 488, Dylight 405 and any combination thereof.
26. The oligonucleotide probe according to aspect 24, comprising at least two labels, wherein one label is a fluorophore located in the terminal 5' or 3' and the other label is a quencher molecule in the opposite terminal.
27. The oligonucleotide probe according to aspect 26, wherein the fluorophore is selected from the group consisting of:
   - 3',6'-dihydroxy-1-oxospiro[2-benzofuran-3,9'-xanthene]-5-carboxylic acid (6-FAM);
   - [2',4,4',5',7,7'-hexachloro-6-[6-[2-cyanoethoxy-[di(propan-2-yl)amino]phosphanyl]oxyhexylcarbamoyl]-6'-(2,2-dimethylpropanoyloxy)-3-oxospiro[2-benzofuran-1,9'-xanthene]-3'-yl] 2,2-dimethylpropanoate (HEX);
   - (2Z)-2-[(3,6-dimethyl-2-phenylpyrimidin-3-ium-4-yl)methylidene]-1-ethylquinoline, chloride (Cy3, Cy5);
   - (3*R*,4*S*,5*S*,6*R*,7*R*,9*R*,11*S*,12*R*,13*S*,14*R*)-6-[(2*S*,3*R*,4*S*,6*R*)-4-(dimethylamino)-3-hydroxy-6-methyloxan-2-yl]oxy-14-ethyl-7,12,13-trihydroxy-4-[(2*R*,4*R*,5*S*,6*S*)-5-hydroxy-4-methoxy-4,6-dimethyloxan-2-yl]oxy-10-(2-methoxyethoxymethoxyamino)-3,5,7,9,11,13-hexamethyl-oxacyclotetradecan-2-one (ROX);
   - 5-chlorosulfonyl-2-(3-oxa-23-aza-9-azoniaheptacyclo[17.7.1.1^{5,9}.0^{2,17}.0^{4,15}.0^{23,27}.0^{13,28}]octacosa-1(27),2(17),4,9(28),13,15,18-heptaen-16-yl)benzenesulfonate (Texas Red);
   - 2-(7-ethyl-3,3,8,8,10-pentamethyl-7-aza-21-azoniahexacyclo[15.7.1.0^{2,15}.0^{4,13}.0^{6,11}.0^{21,25}]pentacosa-1,4(13),5,11,14,16,18,21(25)-octaen-14-yl)-*N*-methyl-*N*-(4-oxopentyl)benzamide (ATTON 647N);
   - (2,5-dioxopyrrolidin-1-yl) 4',5'-dichloro-3',6'-dihydroxy-2',7'-dimethoxy-1-oxospiro[2-benzofuran-3,9'-xanthene]-5-carboxylate (6-JOE);
   - (2*S*,4*R*)-*N*-[(1*S*)-2-methyl-1-[(2*R*,3*R*,4*S*,5*R*,6*R*)-3,4,5-trihydroxy-6-methylsulfanyloxan-2-yl]propyl]-4-propylpiperidine-2-carboxamide (VIC); and
   - (tetrachlorofluorescein): 4,5,6,7-tetrachloro-3',6'-dihydroxyspiro[2-benzofuran-3,9'-xanthene]-1-one (TET).
28. The oligonucleotide probe according to aspect 26 or aspect 27, wherein the quencher is selected from the group consisting of:
   - *N*-[2-[[(*E*)-3-[1-[(2*R*,4*S*,5*R*)-5-[[bis(4-methoxyphenyl)-phenylmethoxy]methyl]-4-[2-cyanoethoxy-[di(propan-2-yl)amino]phosphanyl]oxyoxolan-2-yl]-2,4-dioxopyrimidin-5-yl]prop-2-enoyl]amino]ethyl]-6-[[7-[[2,5-dimethoxy-4-[(4-nitrophenyl)diazenyl]phenyl]diazenyl]-1-azatricyclo[7.3.1.0^{5,13}]trideca-5(13),6,8-trien-6-yl]oxy]hexanamide (BBQ650);
   - 2-[3-(dimethylamino)-6-dimethylazaniumylidenexanthen-9-yl]benzoate (TAMRA);
   - (2S)-2-[4-(3,4-dimethylphenyl)-2-methylquinolin-3-yl]-2-[(2-methylpropan-2-yl)oxy]acetic acid (TQ2);
   - 5-phenylsulfanylquinazoline-2,4-diamine (TQ3); and
   - 2,5-Bis(2-methyl-2-propanyl)-1,4-benzenediol (BHQ-1 and 2).
29. A kit comprising the oligonucleotide probe according to any one of aspects 24 to 28 and/or the oligonucleotide according to aspect 23.
30. The kit according to aspect 29, further comprising at least one forward primer and one reverse primer, wherein the forward primer is SEQ ID NO: 3 and the reverse primer is SEQ ID NO: 5.
31. The use of the oligonucleotide according to aspect 23 or the oligonucleotide probe according to any one of aspects 24 to 28 or the kit according to aspect 28 or 29 for the diagnosis of ulcerative colitis in a subject.
32. The use according to aspect 31, wherein the diagnosis is performed by the method according to any one of aspects 1 to 22.
33. The use of the oligonucleotide according to aspect 32 or the oligonucleotide probe according to any of aspects 24 to 28 or the kit according to any one of aspects 29 or 30 for the detection and/or identification of bacteria associated with ulcerative colitis.

The following examples are provided as merely illustrative and are not to be construed as limiting the scope of the invention.

### EXAMPLES

### Examples - Materials and methods

### Sample collection and storage

Faecal samples: the stool sample was stored in a sterile container and frozen at -25 °C for subsequent extraction of bacterial DNA.

Intestinal biopsies: the samples were stored at 4 °C in sterile tubes with saline solution after collection at the endoscopy unit. In the laboratory, the tissue was washed (3 times) in sterile saline and then, tissue disruption was performed with phosphate buffered saline (PBS) and zirconium spheres (Zirconia/Silica 0.1mm diameter and Minibeadbeater^{™}) for 3 intervals (20, 20 and 30 seconds) at 4800 oscillations/min. The supernatant was then collected in a 1.5 mL tube and centrifuged at 10000g for 5 minutes and the pellet was stored at -25°C for subsequent bacterial DNA extraction.

### DNA extraction

Faecal samples: The commercial kit QIAGEN, QiAamp^{®} DNA Stools Kit-Germany was used. The conditions under the kit were used were those established by the manufacturer except for the last step of the protocol in which distilled water treated with diethylpyrocarbonate (DEPC) was used. The concentration was quantified by Nanodrop and subsequently adjusted to 50 ng/µl in distilled water with DEPC.

Biopsies: The protocol described by Ausubel et al. (Ausubel FM. et al. Short protocols in molecular biology: a compendium of methods from Current protocols in molecular biology. Greene Pub. Associates; 1992) was used. The sample was mixed with 537 µl of TE buffer, 30 µl of 10% SDS and 3 µl of proteinase K (20 ng/ml) and left for one hour at 37 °C. Subsequently, 100 µl of 5M NaCl, 80 µl of CTAB/NaCl was added and incubated at 65 °C for 10 minutes. Subsequently, 750 µl of chloroform/isoamyl alcohol was added and centrifuged at 10000g for 5 minutes. The supernatant was treated with 750 µL of phenol buffer/chloroform/isoamyl alcohol and further centrifuged for 5 minutes. On top of the supernatant extracted in this last step, 450 µL of isopropanol was added and centrifuged again. The isopropanol was removed and 1 mL of 70% ethanol was added and centrifuged again. The ethanol was removed and the precipitate was allowed to dry in the miVac DNA concentrator. To the final product, 30 µL of distilled water with DEPC was added. The concentration was quantified by Nanodrop and subsequently adjusted to 25 ng/µL in distilled water with DEPC.

### Polymerase chain reaction (PCR)

The primers REI-F and REI-R were used, which amplify a region including 93 base pairs of the DNA encoding the bacterial rRNA16S. The sequence of these primers is as follows:
REI-F: CTTGGCCAGCCTTCTTCTGAAAG (SEQ ID NO: 3)
REI-R: CCCCATCGTCTACCGAAAATAC (SEQ ID NO: 5)

PCR with these REI primers included the following conditions and times:
- 2 minutes denaturation at 95 °C
- 35 cycles: 30 seconds at 95 °C followed by 30 seconds at 60 °C and 1 minute at 72 °C.
- Extension for 5 minutes at 72 °C
- The PCR product was stored at 4 °C.

Preparation of the mixture to carry out this PCR in a final volume of 25 µL was as follows: 2.5 U/µL of NZY Proof DNA polymerase high fidelity Taq enzyme from NZYTECH, 0.2 mM dNTPs (NZYSet from NZYTECH), 0.2 µM of each primer and 2.5 µL of the Taq's own buffer.

PCR confirmation was performed using 4% agarose gels (SeaKem^{®}Le Agarose - Lonza) using 3 µL of RealSafe^{™} (iNtRON) and 1X TBE buffer. The marker used was the GeneRuler^{™}100bp Plus DNA Ladder and the electrophoresis time was 40 minutes at 70V.

### Ligation, transformation and cloning

From the PCR, plasmid ligation was performed following the steps indicated in the CloneJet PCR cloning Kit (Thermo Scientific Fermentas), the insert-vector ratio was 3:1 and the 0.5 ml tubes used were of the low binding type. Commercial NZY5a competent E. *coli* cells (NZYTECH) were used for transformation. These cells were mixed with the ligand at a 10:1 ratio in a 1.5 ml tube and left on ice for 30 minutes followed by heat shock at 42 °C for 40 seconds in a heat block. They were then left on ice for 2 minutes and 0.9 mL of SOC culture medium (NZYTECH) was added and left at 37 °C on shaking at 200 rpm for 1 hour. Finally, they were centrifuged at 2400g for one minute and then 700 µL of the supernatant were removed and 200 µL of the resuspended pellet (transformed cells) were seeded on plates with LB Broth medium (Conda-Pronadisa) with 1% agar plus ampicillin (100 µg/mL).

Ten colonies were randomly selected and PCR confirmation of colonies was performed according to the conditions previously described. Cultures were grown in 5 ml of liquid LB with ampicillin (concentration 100 µg/mL) and incubated at 37 °C for 14-16 hours. The commercial QIAGEN kit (QiAprep^{®} Spin Miniprep) was used to extract the plasmids, substituting the recommended buffer with distilled water + DEPC in the last step. After extraction, the plasmid concentration was observed in the Nanodrop and adjusted in the range 30-50 ng/µL.

### Patient characteristics

Seventeen healthy controls and 17 patients with debut UC were included.

Exclusion criteria for this study were: age < 18 years; pregnancy or breastfeeding; inability to understand the study and/or sign informed consent; any malignant disease not considered cured; taking antibiotics or probiotics within the last 30 days; concomitant *Clostridium difficile* or another enteric pathogen infection; severe illness requiring hospitalization; need for any pharmacological treatment that may alter the intestinal microbiota in the previous 3 months; consumption of any experimental drug in the 4 weeks prior to inclusion in the study; any liver disease or malabsorptive disease that can be considered to alter the intestinal microbiota.

The baseline characteristics of the individuals are shown in table 1.

The median age of UC debut in this cohort of patients was 47 years (RIQ 35.3-46.9). Fifty-three percent were men. In terms of smoking habits 71% had never smoked and 24% were ex-smokers with only one patient (6%) being an active smoker at the time of the diagnosis.

Table 1 also shows the endoscopic findings of ulcerative colitis. Regarding the extent of the disease, 41% had involvement of the entire colon, 24% had left colitis and 35% had proctitis. Histological activity was moderate in 82% and severe in 18%.

**Table 1. Patient characteristics at diagnosis**

| | | Healthy Controls n=17 | Ulcerative colitis n=17 | p value |
|---|---|---|---|---|
| Age, years Median (IQR) | | 42.9 (35-43) | 47 (35.3-46.9) | 0.77 |
| Gender, male n (%) | | 9 (52.9) | 9 (52.9) | 1 |
| Smoking habit n (%) | Non smokers | 11 (64.7) | 12 (70.6) | 0.83 |
| | Former smokers | 4 (23.5) | 4 (23.5) | |
| | Current smokers | 2 (11.8) | 1 (5.88) | |
| Location n (%) | Proctitis | N/A | 6 (35.3) | - |
| | Left colitis | N/A | 4 (23.5) | |
| | Pancolitis | N/A | 7 (41.2) | |
| Endoscopic score (Mayo score) | Mayo 1 | N/A | 0(0) | |
| | Mayo 2 | N/A | 12 (70.6) | |
| | Mayo 3 | N/A | 5 (29.4) | |
| Histology | Moderate | N/A | 14 (82.35) | - |
| | Severe | N/A | 3 (17.65) | |

During the first year after diagnosis, 76% of patients were maintained on 5-ASA. Combination treatment with Infliximab + Azathioprine was started in 12%. One patient was started on Infliximab and another on azathioprine in monotherapy.

Patients provided biopsies collected by colonoscopy and a stool sample. Healthy controls provided a stool sample.

### Bacteroidetes genotype

The sequence of genotypes found in this study is shown in table 2.

**Table 2. Sequencing data of the Bacteroidetes genotype (REI primers).**

| Genotype | Sequence |
|---|---|
| N1 (SEQ ID NO: 7) | CTTGGCCAGCCTTCTGAAAGGAAGATTAATCCAGGATGGGATCATGAG TTCACATGTCCGCATGATTAAAGGTATTTTCGGTAGACGATGGGGATC TACTAGTGCGGCCGCCTGC |
| C1 (SEQ ID NO: 8) | CTTGGCCAGCCTTCTGAAAGGAAGATTAATACAAGATGGCATCATGAG TCCGCATGTTCACATGATTAAAGGTATTTTCGGTAGACGATGGGGATC CCGCGGCCATGGCGGCCGG |
| C4 (SEQ ID NO: 9) | CTTGGCCAGCCTTCTGAAAGGAAGATTAATCCAGGATGGCATCATGAG TTCACATGTCCGCAGATTAAAGGTATTTTCGGTAGACGATGGGGATGC GTTCCATTAGATAGTAGGCGG |
| CB1 (SEQ ID NO: 10) | CTTGGCCAGCCTTCTGAAAGGAAGATTAATCCAGGATGGGATCATGAG TTCACATGTCCGCATGATTAAAGGTATTTTCGGTATACGATGGGGATC CCGCGGCCATGGCGGCCGG |
| CB5 (SEQ ID NO: 11) | CTTGGCCAGCCTTCTGAAAGGAAGATTAATCCAGGATGGTATCATGAG TTCACATGTCCGCATGATTAAAGGTATTTTCGGTAGACGATGGGGATC TACTAGTGCGGCCGCCTG |
| CB6 (SEQ ID NO: 12) | CTTGGCCAGCCTTCTGATAGGAAGATTAATCCAGGATGGGATCATGAG TTCACATGTCCGCATGATTAAAGGTATTTTCGGTAGACGATGGGGATC CCGCGGCCATGGCGGCCGG |
| CB7 (SEQ ID NO: 13) | CTTGGCCAGCCTTCTGAAATGAAGATTAATCCAGGATGGGATCATGAG TTCACATGTCCGCATGATTAAAGGTATTTTCGGTAGACGATGGGGATC CCGCGGCCATGGCGGCCGG |
| CB8 (SEQ ID NO: 14) | CTTGGCCAGCCTTCTGAAAGGAAGATTAATGCAGGATGGGATCATGAG TTCACATGTCCCCATGAGTAAAGGTATTTTCGGTAGACGATGGGGATA TAGTAGTGCGGCCGCCTGC |
| CB12 (SEQ ID NO: 15) | CTTGGCCAGCCTTCTGAAAGGAAGATTAATCCAGGATGGGATCATGGG TTCACATGTCCGCATGATTAAGGTATTTTCGGTAGACGATGGGGATCC CGCGGCCATGGCGGCCGG |

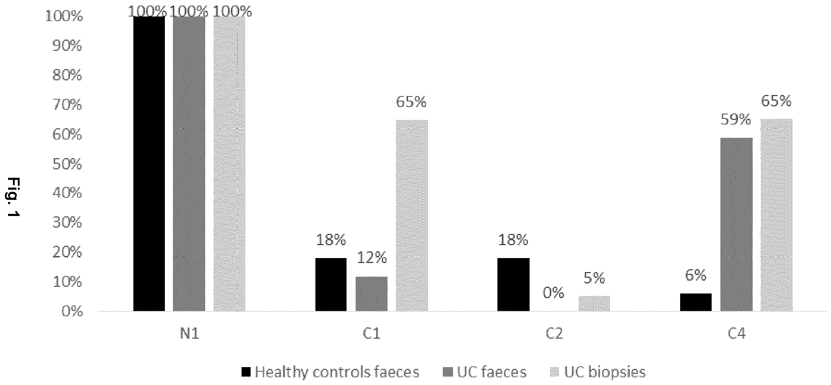

The percentage of occurrence of the different genotypes is summarized in tables 3 and 4 and figure 1. Genotype N1 (*B. dorei*) appeared in 100% of cases and controls in both faecal samples and biopsies. Genotype C1 (*B. vulgatus*) was found in 17.7% of faeces from controls versus 11.8% in faeces from cases, with no significant differences (p=1). The C4 genotype was found in 59% of the stool samples of the patients with significant differences (p= 0.002) when compared to the percentage of occurrence in controls, which was, in this case, 5.9%.

**Table 3. Bacteroidetes genotype in faeces of UC patients and healthy controls**

| | Healthy controls (faeces) n=17 | Ulcerative colitis (faeces) n=17 | p value |
|---|---|---|---|
| Genotype N1 (*B. dorei*) n (%) | 17 (100) | 17 (100) | |
| Genotype C1 (*B. vulgatus*) n (%) | 3 (17.7) | 2 (11.8) | 1 |
| **Genotype C4 n (%)** | **1 (5.88)** | **10 (58.8)** | **0.002** |

In the 17 patients, when comparing the presence of different genotypes found in faeces and biopsies, we observed that there were no significant differences. C4 genotype appeared in 58.8% of the faeces and in 64.71% of the biopsies (0.16) (Table 4).

**Table 4. Bacteroidetes genotype in faeces and biopsies of cases**

| | Ulcerative colitis (faeces) | Ulcerative colitis (biopsies) | P value |
|---|---|---|---|
| Genotype N1 (*B. dorei*) n (%) | 17 (100) | 17 (100) | 1 |
| Genotype C1 (*B. vulgatus*) n (%) | 2 (11.8) | 11 (64.7) | 0.52 |
| Genotype C4 n (%) | 10 (58.8) | 11 (64.7) | 0.16 |

Figure 1 shows the percentage of occurrence of the different predominant genotypes in the samples analysed, showing significant differences in the presence of C4 in both faeces and biopsies compared to healthy controls (p=0.002).

From the sequencing of the C4 genotype, a probe was created that allowed the quantification of C4 in all the samples obtained in study 2 (Table 5).

**Table 5. Sequence of primers and probe C4 used**

| **Name** | **Sequence (5'-3')** |
|---|---|
| BacH_f (SEQ ID NO: 16) | CTT GGC CAG CCT TCT GAA AG |
| BacH_r (SEQ ID NO: 17) | CCC CAT CGT CTA CCG AAA ATAC |
| FGENC4 (SEQ ID NO: 4) | GCC AGC CTT CTG AAA GGA AG |
| RGENC4 (SEQ ID NO: 6) | CCG CCT ACT ATC TAA TGG AAC G |
| SGENC4 (SEQ ID NO: 1) | [FAM] TGG CAT CAT GAG TTC ACA TGT CCG CA [TAM] |

A PCR was carried out with the primers FGENC4/RGENC4 and the following conditions and times:
- 3 minutes denaturation at 95°C
- 30 cycles: 15 seconds at 95°C followed by 15 seconds at 59°C and 45 seconds at 72°C
- extension for 3 minutes at 72°C

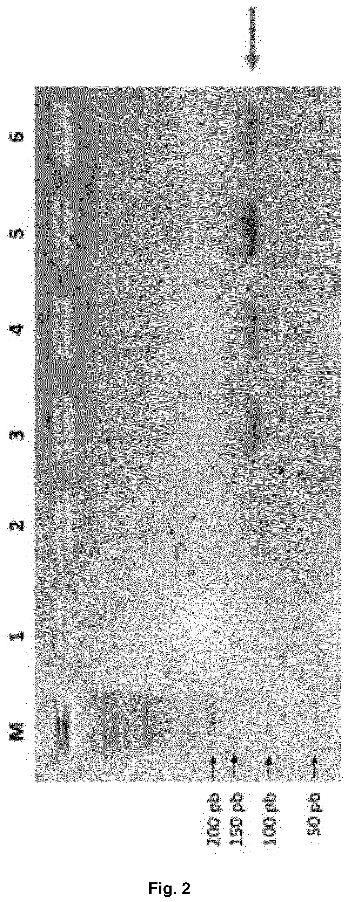

Using a 2% Agarose gel, we visualized the amplicons obtained by PCR with the primers FGENC4/RGENC4 in the DNA extracted from the faeces of a healthy control, a patient with ulcerative colitis and DNA from *Phocaeicola dorei.* Figure 2 shows the results of this gel in which channels 1 and 2 (belonging to DNA extracted from the faeces of healthy controls) do not show any band while the remaining channels 3 and 4 (from the faeces of a patient with UC and 5 and 6 from *Phocaeicola dorei* DNA) show a band of 118 bp.

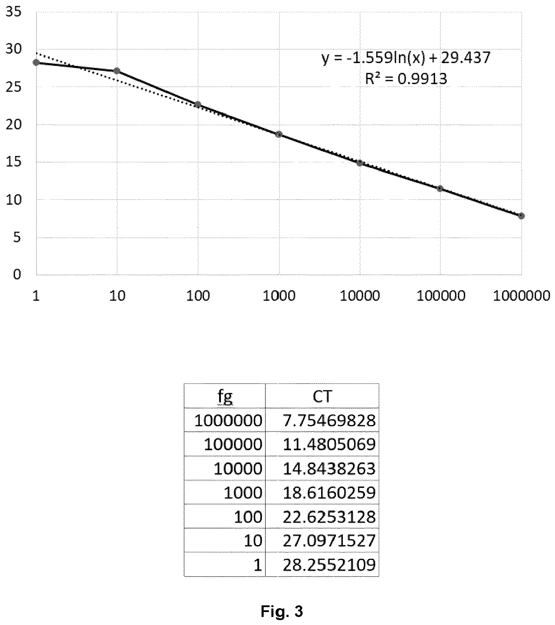

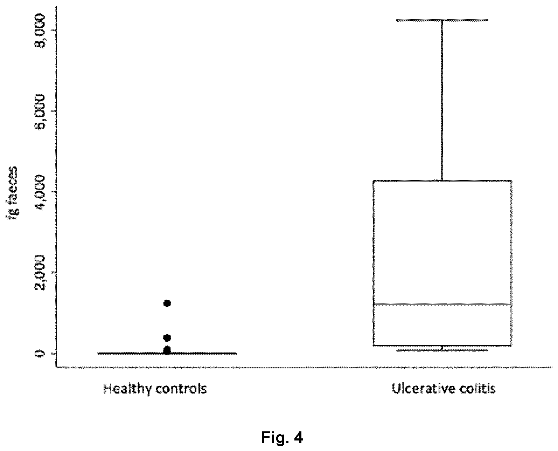

The median qPCR concentration of C4 in faeces from healthy controls was 0.37 fg with a IQR (0.35-4.87) while the median in stool from cases was significantly higher 1218.13 fg (IQR 190.12 - 4278.97) p = (0.001). Regression control line obtained for the qPCR is shown in Figure 3. The qPCR quantification data indicate that stool samples from patients with UC have a higher concentration of Bacteroidetes with the C4 genotype than healthy controls (Figure 4). In biopsies from patients with UC the concentration was 0.67 fg (RIQ 0.11-4.49).

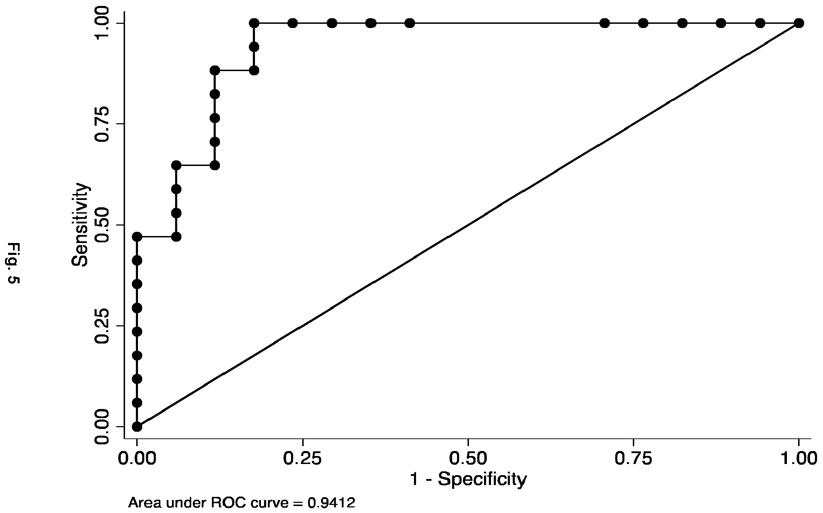

An overall measure of the accuracy of the test for the set of all possible cut-off points was obtained by means of a ROC curve (Figure 5). The area under the curve we obtained was 0.9412. The optimal cut-off point that maximized the overall percentage of correct classifications was 68 fg as it gives a sensitivity of 100%, a specificity of 82%, a positive predictive value of 85% and a negative predictive value of 100%. This high negative predictive value would allow us to rule out UC with high probability.

## Claims

1. An *in vitro* method for the diagnosis of ulcerative colitis in a subject, wherein the method comprises detecting a nucleic acid target region in a sample of feces from the subject, wherein said region comprises the sequence according to SEQ ID NO: 1, the sequence complementary to SEQ ID NO: 1, or a fragment thereof, and wherein the presence of the target region is indicative of the subject having ulcerative colitis.

2. The *in vitro* method according to claim 1, wherein the method comprises the steps of:
(i) submitting the nucleic acids obtained from the sample of the subject to an amplification reaction of the nucleic acid target region obtaining an amplification reaction product wherein the amplification reaction product comprises the nucleotide sequence according to SEQ ID NO: 1 or a fragment thereof and wherein the amplification reaction comprises a forward primer, a reverse primer and an oligonucleotide probe, wherein the forward primer and the reverse primer are specific and capable of amplifying said amplification reaction product, the oligonucleotide probe comprises at least one label capable of emitting a detectable signal and the oligonucleotide probe sequence hybridizes to the nucleotide sequence according to SEQ ID NO: 1 or fragment thereof; and
(ii) detecting the signal emitted by the label during the amplification reaction in step (i),
wherein the detection of the signal emitted by the label is indicative of the subject having ulcerative colitis.

3. The *in vitro* method according to claim 1, wherein the method comprises the steps of:
(i) submitting the nucleic acids obtained from the sample of the subject to an amplification reaction of the nucleic acid target region obtaining an amplification reaction product wherein the amplification reaction product comprises the nucleotide sequence according to SEQ ID NO: 1 or a fragment thereof and wherein the amplification reaction comprises a forward primer and a reverse primer wherein the forward primer and the reverse primer are specific and capable of amplifying said amplification reaction product;
(ii) performing an hybridization reaction of the amplification reaction product with an oligonucleotide probe that hybridizes to the nucleotide sequence according to SEQ ID NO: 1 or a fragment thereof wherein said oligonucleotide probe comprises at least one label capable of emitting a detectable signal, and
(iii) detecting the signal emitted by the label after the hybridization reaction of step (ii),
wherein the detection of the signal emitted by the label is indicative of the subject having ulcerative colitis.

4. The method according to any one of claims 1 to 3, wherein the target region consists of SEQ ID NO: 1.

5. The method according to any one of claims 1 to 4, wherein the target region belongs to *Bacteroidetes* bacteria which comprises in its genome the sequence according to SEQ ID NO: 1 or SEQ ID NO: 2.

6. The method according to any one of claims 2 to 5, wherein the forward primer consists of a sequence selected from the group consisting of SEQ ID NO: 3 and SEQ ID NO: 4.

7. The method according to any one of claims 2 to 6, wherein the reverse primer consists of a sequence selected from the group consisting of SEQ ID NO: 5 and SEQ ID NO: 6.

8. The method according to any one of claims 2 to 7, wherein the oligonucleotide probe consists of a sequence according to SEQ ID NO: 1.

9. The method according to any one of claims 2 to 8 wherein the amplification reaction product comprises a nucleotide sequence with at least 80% identity with a nucleotide sequence according to SEQ ID NO: 2.

10. An oligonucleotide consisting of a nucleotide sequence according to SEQ ID NO: 4 or SEQ ID NO: 6 or a combination thereof.

11. An oligonucleotide probe consisting of a nucleotide sequence according to SEQ ID NO: 1.

12. A kit comprising the oligonucleotide probe according to claim 11 and/or the oligonucleotide according to claim 10.

13. The kit according to claim 11, further comprising at least one forward primer and one reverse primer, wherein the forward primer is SEQ ID NO: 3 and the reverse primer is SEQ ID NO: 5.

14. The use of the oligonucleotide according to claim 10 or the oligonucleotide probe according to claim 11 or the kit according to claim 12 or 13 for the diagnosis of ulcerative colitis in a subject.

15. The use of the oligonucleotide according to claim 10 or the oligonucleotide probe according to claim 11 or the kit according to any one of claims 12 or 13 for the detection and/or identification of bacteria associated with ulcerative colitis.
